Europäisches Patentamt

European Patent Office

Office européen des brevets

(11) Publication number: **0 382 538**
**A2**

# EUROPEAN PATENT APPLICATION

(21) Application number: 90301353.0

(22) Date of filing: 08.02.90

(51) Int. Cl.⁵: **C07K 7/10, A61K 37/64**

(30) Priority: 09.02.89 US 308846

(43) Date of publication of application:
16.08.90 Bulletin 90/33

(84) Designated Contracting States:
CH DE FR GB IT LI NL

(71) Applicant: MERCK & CO. INC.
126, East Lincoln Avenue P.O. Box 2000
Rahway New Jersey 07065-0900(US)

(72) Inventor: Garsky, Victor M.
752 Palmer Place
Blue Bell, PA 19422(US)

(74) Representative: Hesketh, Alan, Dr. et al
European Patent Department Merck & Co.,
Inc. Terlings Park Eastwick Road
Harlow Essex, CM20 2QR(GB)

(54) A process for the preparation of cysteine-rich polypeptides, including echistatin.

(57) A procedure for synthesizing cysteine-rich polypeptides which are inhibitors of platelet aggregation. The procedure minimizes undesired side reactions and intermolecular cross-linking, and can be used to produce the cysteine-rich polypeptides such as:
NH₂-Glu-Cys-Glu-Ser-Gly-Pro-Cys-Cys-Arg-Asn-Cys-Lys-Phe-Leu-Lys-Glu-Gly-Thr-Ile-Cys-Lys-Arg-Ala-Arg-Gly-Asp-Asp-Met-Asp-Asp-Tyr-Cys-Asn-Gly-Lys-Thr-Cys-Asp-Cys-Pro-Arg-Asn-Pro-His-Lys-Gly-Pro-Ala-Thr-OH.

EP 0 382 538 A2

## POLYPEPTIDE SYNTHESIS

## BACKGROUND OF THE INVENTION

Polypeptides having the tripeptide sequence Arg-Gly-Asp and being cysteine-rich were recently derived from vipers, e.g. Trimerersurus gramineus, Echis carinatus, Agkistrodon piscivorus and Bitis arietans. These polypeptides are useful for inhibiting fibrinogen binding to human platelets and inhibiting fibrinogen - induced aggregation of human platelets. They contain a common sequence -Cys-R-R-R-Arg-Gly-Asp-R-R-R-R-R-Cys-, where each R, either the same or different, represents any amino acid.

Synthesis of cysteine-rich peptides is difficult because of the danger of undesirable side reactions, and undesirable intermolecular crosslinking which often occurs during amino acid additions and cleavage of finished peptide from resin.

Echistatin is a single chain polypeptide that is isolated from the venom of the saw-scaled viper Echis carinatus. The primary structure consists of 49 amino acid residues including 8 cysteines. Location of the 4 disulfide bridges in echistatin has not yet been determined. Echistatin contains the tripeptide unit, Arg-Gly-Asp at residues 24-26, a sequence which appears in a variety of adhesive protein ligands and contributes to their interaction with specific membrane receptors Ruoslahti and Pierschbacher (1987) Science Vol. 238, pp. 491-497. For example, the interaction of fibrinogen with its platelet receptor GP IIb/IIIa is likely mediated, at least in part, through one or both of the Arg-Gly-Asp sequences in fibrinogen.

Inhibition of fibrinogen binding to GP IIb/IIIa complex has been shown in animal models to be an effective antithrombotic strategy (Coller (1985) J. Clin. Invest. Vol. 76, pp. 101-108; Gold et al. (1988)- Circulation Vol. 77, pp. 670-677; Yasuda et al, (1988) J. Clin. Invest. Vol. 81, pp. 1284-1291; Collar et al. (1986) Blood Vol. 68, pp. 783-786; Hanson et al, (1988) J. Clin. Invest. Vol. 81, pp. 149-158). Murine monoclonal antibodies directed against GP IIb/IIIa block fibrinogen binding and inhibit platelet aggregation. In canine models these antibodies promote thrombolysis and prevent reocclusion as well as preventing cyclic flow reductions in stenosed coronary arteries. Antibodies to GPIIb/IIIa will also prevent platelet deposition in extracorporeal A-V shunts in a baboon model. Platelet aggregation appears to play a critical role in these models. The monoclonal antibodies are characterized by a prolonged effect on bleeding times and aggregation. Since echistatin has a similar potency to monoclonal antibodies, it would be important to evaluate it in similar animal models of thrombosis.

Several peptides have been chemically synthesized in the size range of echistatin. Nonetheless, the degree of success is dependent on component amino acids. Significant difficulty is encountered during synthesis of peptides having multiple cysteines, particularly with regard to the removal of their protecting groups and the achievement of proper folding. The present invention is a method for the total chemical synthesis of echistatin of sufficient generality to prepare and characterize quantities for in vivo evaluation. Of particular concern was the optimization of folding conditions in the formation of the four disulfide bridges.

## SUMMARY OF THE INVENTION

Echistatin, a polypeptide from the venom of the saw-scaled viper, Echis carinatus, containing 49 amino acids and 4 cystine bridges was synthesized by solid phase methodology in 4% yield. In the final step, air oxidation of the octahydro-derivative was found to be optimal at pH 8. The synthetic product was shown to be physically and biologically indistinguishable from native material. It inhibits fibrinogen-dependent plate let aggregation stimulated by ADP with $IC_{50}$ = 3.3 x $10^{-8}$ M and also prevents aggregation initiated by thrombin, epinephrin, collagen, or platelet activating factor. Reduction of purified synthetic echistatin to octahydroechistatin with DTT followed by air oxidation regenerated homogenous echistatin in quantitative yield. This highly specific refolding strongly suggests that the linear sequence of octahydroechistatin contains all of the information that is required for the proper folding of the peptide. The sequence $Arg^{24}$-Gly-Asp of echistatin, occurs also in adhesive glycoproteins that bind to the platelet fibrinogen receptor - a heterodimeric complex composed of glycoproteins IIb and IIIa (GP IIb/IIIa).

The invention is a process for preparing a cysteine-rich polypeptide containing the following sequence:

X-Cys-R-R-R-Arg-Gly-Asp-R-R-R-R-R-Cys-Y     (I)

wherein X is H or at least one amino acid; Y is OH or at least one amino acid; and each R, either same or different, is any amino acid, wherein

(a) the C-terminal first amino acid of the sequence is attached to a solid cross-linked polystyrene Pam resin to form an amino acyl polymer;

(b) the C-teminal first amino acid blocking group is removed from the amino acyl polymer;

(c) the next sequential second amino acid is coupled to the C-terminal amino acid residue;

(d) subsequent amino acids are coupled in step-wise fashion to form the polypeptide sequence;

(e) the N-terminal Boc group protecting the N-terminal amino acid is removed;

(f) the polypeptide sequence is treated with HF and one or more thio-containing scavengers;

(g) the treated sequence is washed with ether, and transferred to a large volume of dilute acetic acid; and

(h) the solution resulting from step (g) is stirred, and pH adjusted to about 8.0 with ammonium hydroxide.

Polypeptides within the formula I include those of formula Ia:

$H_2N$-(Ch)-Cys-R-R-R-Arg-Gly-Asp-R-R-R-R-R-Cys-(Cx)-H     (Ia)

wherein Ch represents at least one amino acid; Cx represents at least one amino acid; and each R, either the same or different, represents an amino acid.

Preferred embodiments of the invention include processes for preparing polypeptides where Ch represents 1-30 amino acids, more preferably 10-20 amino acids and Cx represents 1-30 amino acids, more preferably 10-20 amino acids. Ch preferably contains 4 cysteines and Cx preferably contains 2 cysteines.

A preferred embodiment of the invention is a process for preparing a polypeptide containing the following sequence:

$NH_2$-Glu-Cys-Glu-Ser-Gly-Pro-Cys-Cys-Arg-Asn-Cys-Lys-Phe-Leu-Lys-Glu-Gly-Thr-Ile-Cys-Lys-Arg-Ala-Arg-Gly-Asp-Asp-Met-Asp-Asp-Tyr-Cys-Asn-Gly-Lys-Thr-Cys-Asp-Cys-Pro-Arg-Asn-Pro-His-Lys-Gly-Pro-Ala-Thr-H

which comprises:

(a) attaching the carboxyl-terminal first amino acid of the sequence to a solid cross-linked polystyrene Pam resin to form an amino acyl polymer, coupling the next sequential second amino acid to the carboxyl-terminal first amino acid, and coupling subsequent amino acids in step-wise fashion until the amino-terminal amino acid is coupled to form the complete polypeptide sequence;

(b) removing amino-terminal BOC group;

(c) cleaving the polypeptide from the resin with HF and oxidizing the polypeptide with a p-thiocresol/p-cresol mixture;

(d) filtering the residue from step(c) and transferring the residue to a solution of acetic acid about 0.4%/$H_2O$ v/v;

(e) stirring the solution and adjusting the pH to about 8.0.


## DETAILED DESCRIPTION OF THE INVENTION

The invention is a procedure for preparing cysteine-rich polypeptides having a common sequence -Cys-R-R-R-Arg-Gly-Asp-R-R-R-R-R-Cys-. The procedure minimizes undesirable side reaction and intermolecular cross-linking, avoids premature loss during deblocking, minimizes sulfoxide formation and thereby provides high yields of the polypeptide.

The polypeptides are generally prepared according to Merrifield, J. Am. Chem. Soc., 85, 2149 (1964), which describes procedures for solid phase synthesis, and according to Stewart and Young "Solid Phase Peptide Synthesis" 2nd Ed., Pierce Chemical Company, Rockford, Illinois, pp. 1-52. The contents of these articles are hereby incorporated by reference. The procedure of the present invention, however, is particularly suited for producing cysteine-rich polypeptides, having platelet aggregation inhibiting properties, in significant yield.


Synthesis of Echistatin

An automatic peptide synthesizer (Applied Biosystems) is used to prepare a polypeptide having the following sequence:

$NH_2$-Glu-Cys-Glu-Ser-Gly-Pro-Cys-Cys-Arg-Asn-Cys-Lys-Phe-Leu-Lys-Glu-Gly-Thr-Ile-Cys-Lys-Arg-Ala-Arg-Gly-Asp-Asp-Met-Asp-Asp-Tyr-Cys-Asn-Gly-Lys-Thr-Cys-Asp-Cys-Pro-Arg-Asn-Pro-His-Lys-Gly-Pro-Ala-Thr-OH

Threonine is attached to a solid cross-linked polystyrene Pam resin using standard solid phase methodology. The Pam resin may be prepared in accordance with the description in Stewart and Young, pp. 11-12. The resin provides excellent peptide-resin link stability during addition of amino acids and protection group removal. The threonine blocking group is then removed from the amino acyl polymer, and alanine is then coupled to the amino acyl polymer. The cycle of deprotection and coupling is repeated with each amino acid that is to be incorporated into the peptide chain. Each amino acid is added stepwise in order to remove the danger of undesirable racemization.

The following blocking groups were used for individual amino acids:

Histidine - benzyloxymethyl
Aspartic Acid - cyclohexyl esters
Glutamic Acid - cyclohexyl esters
Cysteine - 4-methylbenzyl
Serine - benzyl
Arginine - tosyl
Lysine - chlorobenzyloxycarbonyl
Threonine - benzyl
Tyrosine - bromobenzyloxycarbonyl

It is important that double coupling be employed during addition of amino acids.

Critical steps of the procedure of the invention occur after the last amino acid has been added, at which point the finished solution must be treated to obtain proper molecular conformation, and to completely preserve the sequence.

After the last amino acid is added to the sequence, but just prior to cleavage of the polypeptide from the resin, the N-terminal Boc group is removed.

After removal of the protection groups, the resin-peptide is treated with HF. In order to avoid alkylation of residues in the polypeptide, (for example, alkylation of methionine, cysteine, and tyrosine residues) it is necessary to include scavengers in the HF reaction mixture. In accordance with the invention, the scavenger to be used is a thio-containing scavenger, preferably a thio-cresol sand cresol mixture. This scavenger will minimize the oxidation of methionine residues to sulfoxides. After treatment with the above-described HF mixture, the resin is washed with ether, and immediately transferred to a large volume of dilute acetic acid to solubilize and minimize intermolecular cross-linking. A 250 $\mu$M polypeptide concentration is diluted in about 2 liters of 0.1 M acetic acid solution. This procedure contrasts greatly with the procedure which would normally be followed (e.g. where a similar polypeptide concentration would be dissolved in about 50-100 ml of a dilute acetic acid solution). The solution is then stirred and its pH adjusted to about 8.0 using ammonium hydroxide. Upon pH adjustment, the polypeptide takes its desired conformational arrangement.

By following the procedure described in the present invention, one can produce polypeptides having high levels of cysteine residues while minimizing intermolecular cross-linking and undesirable conformational arrangement. In particular, the polypeptide Echistatin is produced in relatively high yields that would ordinarily not be obtained using known methods.

Assembly of the Echistatin protein was achieved by the Merrifield solid phase method Kent and Clark-Lewis (1985) Synthetic Pentides in Biology and Medicine Proc. Labsystems Res. Symp., Eds. Alitalo et al., Elsevier, Netherlands, pp. 29-57; and Merrifield (1963) J. Amer. Chem. Soc. Vol. 85, pp. 2149-2154. Appropriate selection of sidechain protection was made in an effort to minimize the formation of by-products during synthesis and HF deprotection. A double coupling protocol was used for peptide-resin assembly and coupling efficiency monitored by ninhydrin analysis of residual free amine Sarin et al. (1981) Anal. Biochem., Vol. 117, pp. 147-157. Coupling yields were greater than 98.5% in all but three cycles. The additional couplings of Arg[9] and Arg[22] were performed four times and Ile[19] three times. Following these repeated couplings the ninhydrin analysis was improved to a coupling completion level greater than 97%. The final 49-peptide resin which was isolated in a 93% overall yield, was cleaved from the resin using the high HF procedure (Tam et al. (1983) J. Amer. Chem. Soc. Vol. 105 pp. 6442-6455) in the presence of p-cresol and p-thiocresol as scavengers. Use of anisole as a scavenger or the application of the low-high HF procedure (Tam et al. ibid.) proved to be less satisfactory due to the generation of complex and difficult to purify mixtures. Following HF treatment the crude reduced product was subjected to air oxidation in ammonium acetate buffer. Probe oxidations at pH 6.5, 7.0, 7.5 and 8.0 over a period of 4 days indicated the optimum pH to be 8.0. Completeness of reaction was established by Ellman analysis and analytical HPLC. The crude product was purified using preparative HPLC by loading the oxidation reaction directly onto a $C_{18}$-silica reverse-phase column. This technique allowed efficient concentration of 4 liters of solution containing crude echistatin onto the column support followed by a gradient elution for the isolation of the purified product. After a single pass, the product peak was greater than 95% pure. A second gradient elution of the product produced material which was homogeneous by analytical HPLC in the same solvent system in a 4% overall yield. Application of this concerted purification technique enabled us to isolate echistatin with a high level of purity.

The product was characterized for structure and purity. Amino acid analysis after 70 hr acid hydrolysis and performic acid oxidation showed ratios which were within +/- 5% of expected values (Table 1). Sequence analysis of echistatin carried out for 47 cycles gave the expected results. Coinjection of synthetic and native material on analytical reverse-phase HPLC produced a single symmetrical peak. Within the limits of detection (10%) proton NMR in $D_2O$ established the absence of methionine oxide. The NMR also indicated that alkylation of side-chain functionalites had not occurred and was identical with the NMR of a sample of natural product. Amino acid composition, following 70 hr acid hydrolysis, of CNBr treated echistatin was consistant with the expected conversion of methionine to homoserine. This observation further established the absence of methionine sulfoxide. Analysis of the product by SDS polyacrylamide electrophoresis under non-reducing and reducing conditions showed a single peptide band that was indistinguishable from native echistatin.

Examination of the analytical chromotograms during the oxidation of crude echistatin revealed highly efficient folding. In an effort to determine the yield of the oxidation we reduced pure synthetic echistatin to octahydroechistatin with DTT and regenerated homogeneous echistatin in nearly a quantitative yield. The high level of conversion was established by HPLC analysis of the product before and after reduction and reoxidation. Isolation by HPLC of reduced echistatin followed by quantitative Ellman analysis confirmed the product to be in the fully reduced octahydro form. This highly specific refolding shows that the linear sequence of octahydroechistatin contains all of the information that is required for proper folding of the peptide.

In preliminary in vivo studies synthetic echistatin was shown to be a very effective antithrombotic agent.

## EXAMPLE 1

Boc-O-benzylthreonine-PAM resin, Boc protected amino acids and all other reagents required for synthesis on the Applied Biosystems 430 A (ABI) peptide synthesizer were obtained from the manufacturer. Sidechain protected Asp, Glu and His were supplied by Bachem, Inc. The solvents dimethylformamide (DMF) and methylene chloride ($CH_2Cl_2$) were obtained from Burdick and Jackson. Dithiothreitol (DTT) was purchased from Bethesda Res. Labs. Dithioerythritol (DTT) was obtained from Chemical Dynamics. p-cresol and p-thiocresol were obtained from Aldrich Chemical Co.

### Echistatin Synthesis

Starting with 0.50 mM (0.69 g) of Boc-Thr(Bzl)O-Pam-resin (substitution at 0.72 mM of Thr/g of resin) the synthesis was carried out in a stepwise manner using the ABI automated peptide synthesizer. Kent and Clark-Lewis (1985) Synthetic Peptides in Biology and Medicine, Proc. Labsystems Res. Symp., Eds., Alitalo et al., Elsevier, Netherlands pp. 29-57. The amino acids were introduced using the manufacturer's prepacked cartridges (2 mM each). Sidechain protection was Arg (Tos), Asp (OcHx), Cys (Meb), Glu (OcHx), His (Bom) Lys[Z(Cl)], Ser (Bzl), Thr (Bzl), Tyr[Z(Br)]. [Tos, Tosyl; cHx, cyclo-hexyl; Meb, 4-methylbenzyl; Z(Cl), 2-chlorobenzyloxycarbonyl; Bom, benzyloxymethyl; Bzl, benzyl; Z(Br), 2 bromobenzyloxycarbonyl]. Double coupling with symmetric anhydrides (performed in $CH_2Cl_2$ followed by solvent exchange with DMF) were used for all Boc-protected amino acids except for Arg (Tos), Asn and His (Bom), where hydroxybenzotriazole esters in DMF were used in a double coupling protocol. In order to protect against undesired acid-catalyzed oxidations of Cys and Met (Draper et al. (1973) J. Med. Chem. Vol. 16, pp. 1326-1329) during trifluoroacetic acid (TFA) deblocking, 0.1% (wt/v) DTE was added as a scavenger. Following the coupling of N-terminal Glu the Boc group was removed using TFA and the peptide-resin was dried. The final weight of N-terminal deblocked peptide-resin was 4.15 g.

### HF Cleavage and Oxidation

The assembled peptide-resin (2.0 g) was suspended in a mixture of 3 ml of 1:1 (v:v) p-thiocresol/p-cresol in an HF apparatus (Peninusula Labs. Inc., Type 1B). The system was evacuated with a mechanical vacuum pump and HF condensed (30 mL) using liquid nitrogen cooling. After stirring at 0-5° C for 1 1/2 hr the reaction mixture was evaporated in vacuo using a liquid nitrogen trap (20-30 min). The residue was triturated with ether, filtered and washed 3 times with additional ether. The filtered residue was immediately transferred to 4 liters of a stirred solution of dilute acetic acid (0.4%/$H_2O$). After stirring for several minutes the pH of the mixture was adjusted to 8.0 with ammonium hydroxide. Following filtration to remove resin, the crude oxidation product was maintained without stirring at 5°C (18 hr) and subsequently at ambient temperature (19-20°C) for 3 days. Analytical HPLC was used to monitor the progress of the oxidation. A qualitative Ellman test (Habeeb, Methods in Enzymology (1972), Eds. Hirs and Timasheff, Academic Press New York pp. 457-464) was used to monitor the disappearance of free sulfhydryl groups before proceeding with purification. This test was performed on a 1 ml sample which was lyophilized in order to remove residual p-thiocresol.

## Purification of Crude Oxidized Echistatin

The crude oxidized solution (4 l) was acidified by addition of acetic acid (10 ml) and pumped directly onto a $C_{18}$-silica (5 x 30 cm, 15$\mu$, 300 A) cartridge (Waters Associates). The product was purified using preparative HPLC (Separations Technology, Inc.). A step gradient (100 mL increments) which was generated from 1 liter each of successively increasing concentrations of mobile phase. A flow rate of 70 mL min was used to elute the product. Homogenous (>95%) fractions as determined by RP-HPLC (Vydac $C_{18}$, 218TP5415) were pooled and lyophilized to give 72 mg of product. The semi-pure product was contaminated with a less polar component as a shoulder to the product peak. The product was further purified by repassage on HPLC in the same manner as described above to yield echistatin (54 mg). Based on 0.25 mM of starting resin this weight represents a 4 percent overall yield. Homogeneity was demonstrated by analytical HPLC. Coinjection of synthetic product with native material gave a single peak. Product was further characterized by amino acid analysis after hydrolysis with 6N HCl (Table 1) and by automated Edman degradation (ABI 470A Protein Sequencer).

TABLE 1

| AMINO ACID ANALYSIS[a] OF SYNTHETIC ECHISTATIN (THEORETICAL NUMBER IN PARENTHESIS) | | | |
|---|---|---|---|
| Lys | 5.00 (5) | Gly | 5.03 (5) |
| His | 1.00 (1) | Ala | 2.10 (2) |
| Arg | 3.87 (4) | Cys[d] | 7.67 (8) |
| Asp | 8.13 (8) | Met[c] | 0.84 (1) |
| Thr[b] | 2.99 (3) | Leu | 0.98 (1) |
| Ser[b] | 1.04 (1) | Phe | 0.95 (1) |
| Glu | 3.07 (3) | Tyr | 1.00 (1) |
| Pro | 4.08 (4) | Ile + allo-Ile | 0.99 (1) |

[a]After hydrolysis with 6N HCl at 100°C for 70 hrs.
[b]Corrected for decomposition during hydrolysis.
[c]Uncorrected.
[d]Determined as cysteic acid following performic acid oxidation.

A maximum of 1.9 percent preview was observed. The high yield of PTH amino acids from the first step also demonstrated that cyclization of the N-terminal Glu to pyro-Glu had not occurred.

## Reduction and Refolding of Synthetic Echistatin

Synthetic echistatin (0.50 mg) was dissolved in 1 ml of 0.07 M pH 8.0 ammonium acetate (10 mM DTT) and the course of reduction followed by analytical HPLC. After 1 hr the starting material was converted quantitatively to a single reduced product. Dialysis (24 hr) of the reduced product using a 12 mm diameter, 1000 MW cutoff, cellulose tubing (Spectrum Medical Ind.) against (4 liters) of a 0.07 M ammonium acetate buffer (pH 8.0) produced only echistatin. In order to demonstrate that the echistatin was in its fully reduced

6

form prior to reoxidation, the DTT reduction of synthetic echistatin was repeated as described but in the presence of 6M guanidine hydrochloride. Analytical HPLC confirmed that the reduced products had identical times. Isolation of reduced echistatin, by semi-preparative HPLC, followed by quantitative Ellman analysis (Habeeb, ibid.) showed the product to be in the octahydro form.

## Platelet aggregation assay

Platelet aggregation was measured at $37^{\circ}$C in a Chronolog aggregometer. The reaction mixture contained washed platelets ($2 \times 10^8$/ml), fibrinogen (100 $\mu$g/mL), $Ca^{2+}$ (1 mM), and the platelet aggregation inhibitor fraction to be tested. The aggregation was initiated by adding 10 $\mu$M ADP 1 min after the other components had been added. The reaction was allowed to proceed for at least 2 min. The extent of inhibition of aggregation was expressed as the percentage of the rate of aggregation observed in the absence of inhibitor. Synthetic echistatin possesses chemical and biological properties indistinguishable from those of natural echistatin (Table 2).

TABLE 2

| EFFECT OF NATIVE ECHISTATIN AND SYNTHETIC ECHISTATIN ON INHIBITION OF HUMAN PLATELET AGGREGATION | |
|---|---|
| PEPTIDE | $IC_{50}(\mu M)$ (95% CONFIDENCE LIMITS) |
| Native Echistatin | 0.032 (0.026-0.039) |
| Synthetic Echistatin | 0.033 (0.026-0.041) |

The concentration of peptide necessary to inhibit the rate of human platelet aggregation by 50% ($IC_{50}$) was determined using platelets from 2-4 donors. Determinations were performed in duplicate for each donor.

Polypeptides prepared by the synthesis of the invention may be administered in any situation where inhibition of human or mammalian platelet aggregation is desired.

These polypeptides are useful in surgery on peripheral arteries (arterial grafts, carotid endarterectomy) and in cardiovascular surgery where manipulation of arteries and organs, and/or the interaction of platelets with artificial surfaces, leads to platelet aggregation and consumption. The aggregated platelets may form thrombi and thromboemboli. The polypeptides may be administered to these surgical patients to prevent the formation of thrombi and thromboemboli.

Extracorporeal circulation is routinely used for cardiovascular surgery in order to oxygenate blood. Platelets adhere to surfaces of the extracorporeal circuit. Adhesion is dependent on the interaction between GPIIb/IIIa on the platelet membranes and fibrinogen adsorbed to the surface of the circuit. (Gluszko et al., Amer. J. Physiol., 1987, 252:H, pp. 615-621). Platelets released from artificial surfaces show impaired hemostatic function. Polypeptides synthesized according to the process of the invention may be administered to prevent adhesion.

Other applications of these polypeptides include prevention of platelet thrombosis, thromboembolism and reocclusion during and after thrombolytic therapy and prevention of platelet thrombosis, thromboembolism and reocclusion after angioplasty of coronary and other arteries and after coronary artery bypass procedures. These polypeptides may also be used to prevent myocardial infarction.

The synthesized polypeptides may be administered by any convenient means which will result in its delivery into the blood stream in substantial amount. They may be combined with other platelet aggregation inhibitors such as plasminogen activators in order to inhibit platelet aggregation. Intravenous administration is presently contemplated as the preferred administration route. They are soluble in water, and may therefore be effectively administered in solution.

In one exemplary application, a suitable amount of the polypeptide is intravenously administered to a heart attack victim undergoing angioplasty. Administration occurs during or several minutes prior to angioplasty, and is in an amount sufficient to inhibit platelet aggregation, e.g. an amount which achieves a steady state plasma concentration of between about 0.05 - 20 μM.

## Claims

1. A process for preparing a cysteine-rich polypeptide containing the following sequence:

H₂N-(Ch)-Cys-R-R-R-Arg-Gly-Asp-R-R-R-R-R-Cys-(Cx)-H

wherein Ch represents at least one amino acid; Cx represents at least one amino acid; and each R, either the same or different, represents an amino acid,

which comprises:

(a) attaching the carboxyl-terminal first amino acid of the sequence to a solid cross-linked polystyrene Pam resin to form an amino acyl polymer, coupling the next sequential second amino acid to the carboxyl-terminal first amino acid, and coupling subsequent amino acids in step-wise fashion until the amino-terminal amino acid is coupled to form the complete polypeptide sequence;

(b) removing the amino-terminal BOC group;

(c) cleaving the polypeptide from the resin with HF and oxidizing the polypeptide with a p-thiocresol p-cresol mixture;

(d) filtering the residue from step (c) and transferring the residue to a large volume of 0.4% $H_2O$ v/v acetic acid solution; and

(e) stirring the solution and adjusting the pH to about 8.0.

2. A process of claim 1 for preparing a polypeptide containing the following sequence:

NH₂-Glu-Cys-Glu-Ser-Gly-Pro-Cys-Cys-Arg-Asn-Cys-Lys-Phe-Leu-Lys-Glu-Gly-Thr-Ile-Cys-Lys-Arg-Ala-Arg-Gly-Asp-Asp-Met-Asp-Asp-Tyr-Cys-Asn-Gly-Lys-Thr-Cys-Asp-Cys-Pro-Arg-Asn-Pro-His-Lys-Gly-Pro-Ala-Thr-H